(19) Europäisches Patentamt
European Patent Office
Office européen des brevets

(11) EP 1 488 815 A1

(12) EUROPEAN PATENT APPLICATION

(43) Date of publication:
22.12.2004 Bulletin 2004/52

(51) Int Cl.[7]: A61L 15/46, A61L 15/26, A61L 15/42

(21) Application number: 03076909.5

(22) Date of filing: 18.06.2003

(84) Designated Contracting States:
AT BE BG CH CY CZ DE DK EE ES FI FR GB GR HU IE IT LI LU MC NL PT RO SE SI SK TR
Designated Extension States:
AL LT LV MK

(71) Applicant: Corpura B.V.
4041 CL Kesteren (NL)

(72) Inventor: Verweire, Ineke
B-9112 Sinaai-Waas (BE)

(74) Representative: Van Reet, Joseph et al
Gevers & Vander Haeghen,
Intellectual Property House,
Brussels Airport Business Park
Holidaystraat 5
1831 Diegem (BE)

(54) **Antimicrobially effective medical dressings and methods for their preparation**

(57) The invention provides an antimicrobially active polymeric flexible material suitable for use in body care applications and a wound dressing comprising such an antimicrobially active material. This material comprises a polymeric substrate having covalently bonded thereto at least one antimicrobially active compound, in particular a quaternary ammonium compound comprising an apolar end chain for penetrating into the cell membrane and a reactive siloxane group. The material according to the invention is hydrophilic and has a relatively high fluid absorption capacity both after saturation of the material and after draining and a relatively small wet out. The wound dressing thus keeps the wound wet, without leaching any antimicrobial agent in the wound, and can be left for quite a long time onto the wound.

EP 1 488 815 A1

## Description

The invention relates to wound dressings and particularly but not exclusively to such dressings that are useful in the treatment for heavily exuding wounds.

In advanced wound care, it is believed that wet wound care has significant advantages. An ideal wound dressing should have following characteristics:

1) remove excess exudate away from the wound surface
2) maintain high humidity at the wound dressing interface to promote healing
3) provide thermal insulation and cushioning of the wound
4) allow gases exchange and the passage of water vapour
5) not leave dressing residues as polymeric particles or fibres, nor leach out toxic substances
6) be impermeable to micro-organisms
7) not adhere to new tissue formed in the wound
8) not cause pain nor trauma during the exchange of the dressing

Already a lot of dressings are commercially available that try to fulfil all these requirements. Different materials such as hydrocolloids, alginates, hydrogels and hydrophilic foams, membranes and barrier films, or combination of these materials are described in the prior art. However, these dressings do not affect possible present infections or bacterial contaminations occurring during dressing exchange. Deep and open wounds are extremely susceptible to infection, because of the lack of a skin barrier and the excess of food the wound offers to bacteria. Moreover, in the hospital environment, the risk to be infected with bacteria which are resistant to antibiotics increases significantly. Infected wounds heal very difficult and are very difficult to treat.

In common, infected wounds are locally treated with antimicrobially active agents, applied directly in a pure, dissolved or emulsified form to the wound and mostly covered with a gauze or advanced dressing. Examples of such antimicrobial agents are mercury agents such as thimerosal (Merthiolate®) or merbromin (Mercurochrome®) or silver compounds as silver salts (f.i. silvernitrate 0.5-1% solutions), silver sulfadiazine complex (f.i. trademarks Silvadene® and Flamazine®, or iodine tincture ( as isobetadine®).

However there are certain disadvantages applying these agents directly to the wound:

- they show some cytotoxic reactions, due to the toxicity of the agent or the compound components,
- they can attack and destroy cells and substances in the wound fluid that promote wound healing whereby the wound healing process is slowed down or even inhibited
- high concentrations are needed causing acquired resistance and/or allergic reactions
- they are often unstable in light
- they are difficult to handle and
- they need to be applied several times a day.

In order to avoid these negative effects, dressings have been developed, having one or more incorporated antimicrobial agents.

Antiseptics based on the silver ion are further used in various medical devices (f.i. Actisorb® of J&J, Avance® of SSL international), where the antibacterial activity of the silver compound is combined with the active wound exudate management of the dressing. In the WO 02/062403 (Coloplast), a medical dressing is described, comprising an antimicrobial silver complex and being capable of releasing the silver ion into the wound.

In WO99/13923 (Tyco group) a hydrogel wound dressing is described, which may comprise a bacteriostatic and or antimicrobial agent, particularly bismuth tribromophenate or particularly silversulfadiazine. All these agents are released in the wound.

Dressings having a releasable antimicrobial agent may have adverse effects on the wound healing process, because of toxic reactions to healthy cells or influencing the activity of healing body agents or inflammation reactions as a result of eliminating the antimicrobial agent residues. Moreover there is a need to renew periodically the dressing to maintain its antimicrobial properties.

Various attempts have been made to overcome these deficiencies by bonding the active antimicrobial substance to the dressing.

US-A-6160196 (Beiersdorf) discloses a hydrophobic wound covering, consisting of a synthetic or natural polymeric fiber material, having physically adhered or bonded thereto an antimicrobially active compound. The bacteria will be adsorbed by the hydrophobic wound dressing and are destroyed on the wound covering. This covering is however not suitable for heavily exuding wounds and, when an absorption of wound exudate is desired, the covering has to be perforated and an absorbent layer has to be deposited on the covering.

In US-A1-2002/0177828 a medical dressing is disclosed having a non-leaching antimicrobial coating applied to a gauze-type substrate. The coating is covalently bonded to the substrate by a graft polymerisation of non-hydrolyzable allyl or vinyl containing monomers, having preferably quaternary ammonium groups. The method for the preparation of this dressing composition however comprises several process steps, making this process time consuming and expensive.

In US-A- 4 631 297 (Dow Corning), antimicrobial effective foams are disclosed, using certain silanes as antimicrobial agent. In a one step process a foamable organic system is mixed with an organosilane. Due to the covalent reaction with the reactive components in this foamable system, the antimicrobial component will not leach. The described foams are however not hydrophilic, neither suitable for medical use, because of the use of toxic leachable catalysts and surfactants.

It is the aim of the invention to overcome the prior art problems by providing new and improved antimicrobially active polymeric flexible materials, in particular foams, suitable for use in body care applications, such as wound care, incontinence care, ostomy care, skin care and cosmetic care, and in particular wound dressings comprising such a material according to the invention. These wound dressings may be self-adhering or non-adhering.

The antimicrobially active polymeric flexible material according to the invention is self-adhering or non-adhering and comprises a polymeric substrate having covalently bonded thereto at least one antimicrobially active compound. The polymeric substrate is hydrophilic, able to manage the wound exudate of highly exudating wounds. The polymeric substrate or constituent may preferably be a separate element of an absorbing foam, a hydrogel, a hydrocolloid and/or an alginate or a combination of two or more of these materials. Alternatively, particulates or fibers of the above mentioned polymeric constituents may be distributed, preferably homogeneously, in or onto a base dressing substrate as a gauze, a non-woven or a polymer film.

It has been found suitable that the fluid absorption capacity after saturation of the antimicrobially active material of the invention is higher than 4 grams per gram material, preferably higher than 8 grams, preferably higher than 12 grams, and most preferably higher than 15 grams per gram dry material.

Because it is important that the wound dressing retains the wound exudate during treatment of removal of the wound dressing, it has been found suitable as well that the fluid absorption capacity after draining of the polymeric substrate of the wound covering is higher than 2, preferably higher than 6 grams, preferably higher than 10 grams, and most preferably higher than 13 grams per gram dry material.

To avoid leaching of the excess of exudate at the side borders of the wound covering due to a slow absorption of the exudate, which subsequently may cause maceration of the surrounding skin, it has been found suitable that the wet out, this is the absorption velocity in seconds of a drop of 1 ml of water in the middle of the material, is lower than 200 s, preferably lower than 100 s, more preferably lower than 50 s and most preferably lower than 10 s.

In a preferred embodiment of the invention, the antimicrobially active material comprises a polyurethane foam layer, forming preferably at least a part of the skin-contacting surface of a wound dressing.

The hydrophilic polyurethane foams are made based on the technology of hydrophilic polyurethane prepolymers. The prepolymer which is used in the method of the invention is preferably an isocyanate-capped polyether, such as an polyethyleneoxide or an ethyleneoxy/propyleneoxy copolymer. A particularly suitable prepolymer is described in GB-A-1 429 711 and in WO96/16099 or US6271277. Suitable prepolymers are commercially available under the Trade Mark HYPOL (Dow Chemical) or as Optipol (LMI).

When the polymeric substrate is a foam, this foam has preferably an open-celled, very fine cell structure with an average cell diameter in the range of 100 to 800 microns, since such foams are found to be appropriate for low adherence wound dressings.

To prepare the antimicrobially active material of the invention, an antimicrobially active agent will be covalently bonded to the foam. This antimicrobially active agent preferably consists of a quaternary ammonium compound, having an apolar end chain and a reactive group that can provide a covalent bond with the polymeric substrate. Suitable antimicrobial active agents are disclosed in US-A-4 631 297 (Dow Corning), wherein the reactive antimicrobial agent is an organosilane, having the general formula:

$$(RO)_3\ Si\ (R')\ (R'')N^+\ (R''')(R'''')\ X^-$$

or

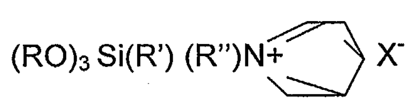

These antimicrobially active agents are available under the Trade Mark AEGIS (Devan Chemicals)

Examples of other reactive groups of the antimicrobially active agent may be, but are not limited to isocyanates, epoxides, vinyl or allyl monomers, substances that have the capability to form radicals and to polymerise under exposure of initiator molecules, UV or other sources of radiation.

The antimicrobial activity of the preferred antimicrobial of the invention is based on the disruption of the cell membrane of the targeted micro-organisms. The interruption is caused by two mechanisms: the long apolar chain group physically perforates the cell membrane, and the positively charged nitrogen component interferes with the ionic transport fluxes through the cell membranes. Due to these physical interactions, the targeted micro-organisms are not able to build up resistance against the antimicrobially active agent, which is a well-known effect and discussed issue in antibiotic treatment of infections.

The antimicrobially active agent can be incorporated in the polymeric structure, after the formation thereof, by impregnation, spraying, dipping, brushing, padding, exhaustion, or other foam finishing techniques. In order to achieve a more uniform distribution of the antimicrobially active agent, it is preferably incorporated in the polymeric substrate during the formation thereof, in particular during the foaming.

The objective of the wound dressing of the invention is to provide an antimicrobial wound dressing, showing a good absorbing capacity, as well as a durable and enhanced antimicrobial effectiveness. It is also an objective that the active antibacterial component is entirely immobilized in the substrate so that it does not leach into the environment and so that it is stable during the lifetime of the dressing. This avoids any negative reactions at the wound interface.

US-A-4 631 297 (Dow Corning) discloses concentrations of the antimicrobial agent of between 1 and 5 parts per 100 parts polyol, the lowest concentrations causing however a substantially smaller reduction of the number of bacteria when measuring this reduction according to the shake flask method ASTM E 2149-01.

Surprisingly, it has been found according to the present invention that an important bactericidal activity was already obtained with smaller amounts of the antibacterial agent than described in US-A-4 631 297 when using a substrate that is more hydrophilic than the substrates disclosed in this US patent. The present inventors have incorporated for example the antimicrobial agent Aegis 5772 into a hydrophilic foam, during the production thereof starting from an isocyanate prepolymer, in amounts of respectively 0 parts (A), 0.6 parts (B), 0.4 parts (C), 0.2 parts (D) and 0.1 parts by weight (E) agent per 100 parts by weight dry foam substrate. The results in Table I show that concentrations as low as 0.2 parts agent per 100 parts dry foam substrate surprisingly still showed a 99.99% reduction of micro-organisms, in particular of bacteria. Further optimalisation may reduce the required amount of antimicrobial agent to a value between 0.2 and 0.1 parts per 100 parts dry foam. Table I: Effect of the incorporation of different parts by weight of Aegis 5772 on the reduction of E. coli and Aspergillus niger and on the distribution of this antimicrobial agent in a hydrophilic polyurethane foam into which it is incorporated during the formation thereof.

| | Parts Aegis 5772 | Parts Lurol 8778 | Microbiological analysis (% reduction of Escherichia coli) | Fungal analysis Growth rating Aspergillus niger | Chemical analysis Homogeneous distribution Bromophenol Blue colouring |
|---|---|---|---|---|---|
| A | 0.0 | | 0.0 | 4 | Homogenous slightly blue |
| B | 0.6 | | 99.99 | 0 | Dark blue spots |
| C | 0.4 | | 99.99 | 0 | Dark blue spots |
| D | 0.2 | | 99.99 | 0 | Homogeneously blue |
| E | 0.1 | | 0.0 | 2 | Homogeneously blue |
| | | | | | |
| F | 0.6 | 0.3 | 99.99 | 0 | Homogeneously blue |
| G | 0.4 | 0.2 | 99.99 | 0 | Homogeneously blue |
| H | 0.4 | 0.5 | | | |

Moreover and even more surprisingly, these same low concentrations showed effective antifungal activity as well. The antibacterial and the antifungal activity was tested according to the AATCC30 method, more particularly against the bacterium Escherichia coli and the fungus Aspergillus niger.

Surprisingly as well, the antimicrobially active materials made in accordance with the invention, preferably hydrophilic foam dressings, showed a high antibacterial activity, although the antimicrobial agent is not leaching and although they show highly hydrophilic properties. Tabel II shows the average values of the hydrophilic characteristics of the tested dressings.

Table II:

| Physical parameters of the foams indicated in Table I. | | | | | | | | |
|---|---|---|---|---|---|---|---|---|
| | A | B | C | D | E | F | G H | |
| Cell size (p) | 100-220 | 300-400 | 300-400 | 300-400 | 300-400 | 300-400 | 300-400 | 250-350 |
| Wet out (s) | <1 | 215 | 780 | <1 | <1 | 6 | 4 | 2 |
| Fluid capacity saturated (g water/ g dry material) | 22 | 16 | 16 | 20 | 23 | 15 | 16 | 18 |
| Fluid capacity after draining (g water/g dry material) | 21 | 8 | 8 | 13 | 22 | 8 | 8 | 15 |
| +% elongation dry | 271 | 178 | 187 | 203 | 255 | 176 | 176 | 238 |
| Strength at break, dry(kPa) | 86 | 67 | 72 | 70 | 102 | 73 | 73 | 117 |

In comparison with foam A (blank foam), foam samples B and C show a significantly increased wet out, as well as a decreased fluid capacity saturated and a decreased fluid capacity after draining. This proves that the antimicrobial agent renders the foam more hydrophobic. The reason why the wet out of foam C was higher than the wet out of foam B is due to the unhomogenous distribution of the antimicrobial active agent. This was proved with the bromophenol blue colouring test indicating locally higher concentrations of the agent in foam B and C (see Table I). Probably if higher concentrations of the antimicrobial active agent, in this case Aegis 5772, are used, the reactive siloxane group reacts more quickly with other siloxane groups than with chemical groups of the substrate material.

It was also found that addition of the antimicrobial agent may reduce the mechanical properties of the foam as shown in Table II. Surprisingly, this negative effect could be eliminated by reducing the concentration of the antimicrobial agent, as shown for Foam D, or by adding a hydrophilising additive as shown for foam F, G, H. In this case a polyether silicone derivative was used, known under the trade name Lurol 8728 (Devan Chemicals). Concentrations of 0.3 parts (F), 0.2 parts (G), and 0.5 parts by weight (H) Lurol 8728 per 100 parts by weight dry foam substrate were used.

Without limiting the invention to any specific theory it is assumed by the present inventors that the microorganisms are drained away from the wound site, together with the wound exudate. After contacting the wound dressing surface they will die out and microorganism residues will be removed from the wound site with exchange of the dressing.

In addition to the covalently bonded antimicrobial agent, the wound dressing of the invention may also include topical medicaments and other leachable antiseptics, as well as other therapeutically useful additives such as polypeptides, growth factors and enzymes.

The wound dressing or antimicrobially active flexible material may show whether or not adhesive properties. In the last case it will then typically be secured on the desired wound site using conventional means such as a cover dressing.

In a preferred embodiment of the invention, the dressing comprises a skin-contacting surface comprising an area showing a skin friendly adhesive. Such a dressing may also suitably comprise a water-impervious backing layer, film or membrane whereto a skin-friendly adhesive is applied, to which the absorbing, antimicrobial polymeric substrate is applied, forming an island dressing. The water impervious layer, film or membrane may be of any suitable material known in the preparation of wound dressings as a non-woven layer, a foam, a polyurethane, polyethylene, polyester or polyamide film. The skin friendly adhesive may exist of adhesive hydrocolloids or medical grade acrylate based adhesives.

Moreover, in a preferred embodiment of the invention, a water based, medical grade acrylate based adhesive is applied during formation of the hydrophilic polyurethane foam substrate, that may comprise a covalently bonded

antimicrobial agent. Surprisingly to all other methods this can be done in a 1 step process. After the foam is blown up, a medical grade, water based adhesive is laid down on the curing foam. Subsequently the polymeric substrate and the adhesive layer is dried. A suitable water based adhesive is Aqualine PSA 1623 (Collano). All known methods, such as but not limited to reverse roll coating, kiss coating, flat die system or rotary screen printing can be used. Most preferably a dot screen printing is applied. All kind of patterns can be applied, but preferably a dot with a diameter between 0.4 and 4 mm, preferably between 1 and 1.5 mm, with a distance between the dots varying between 0.3 and 8 mm, and preferably between 0.5 and 2.5 mm. This guarantees that the hydrophilic properties of the polymeric foam substrate are maintained (see table III). The total area of the polymeric foam substrate surface can be covered with adhesive. However, more preferably only the side borders, aimed to cover healthy skin, are coated with adhesive dots. To obtain sufficient adherence strength, that allows normal movement of the body parts, but does not cause trauma or pain at removal of the dressing, adhesive amounts between 25 $g/m^2$ and 300 $g/m^2$ are applied, preferably between 50 $g/m^2$ and 250 $g/m^2$, and more preferably between 100 $g/m^2$ and 200 $g/m^2$.

Table III:

| Hydrophilic properties of a hydrophilic polyurethane foam having applied thereon dots of an adhesive. | | |
|---|---|---|
| | Foam without adhesive | Foam with screen print dot application of adhesive aqualine PSA 1623 |
| Wet out (s) | 4 | 10 |
| Fluid capacity after 3 min (g/g) | 16.3 | 21.4 |
| Fluid capacity after 30 min (= saturated) (g/g) | 21.4 | 21.4 |

The invention further relates to a one step, continuous method for preparing a wound covering, comprising preferably a hydrophilic foam substrate, comprising the steps of:

1) intensively premixing a reactive antimicrobial agent with a component stream A which is preferably an aqueous stream, comprising surfactants and/or any other active fillers;
2) mixing the A stream with the other product stream or streams, preferably a stream with one or more isocyanate prepolymers, but it is contemplated that such a foam system may also be produced directly from a separate polyol and isocyanate stream, additionally with catalysts, stabilisers or colours streams;
3) transforming the mixture into a thin layer having a predetermined thickness;
4) if desired, applying a water based adhesive;
5) drying and curing, preferably with microwave technology, the thin layer; and
6) converting the thin layer to the final dressing design.

Instead of mixing the reactive antimicrobial agent first with component stream A, it can also be supplied in a separate stream which is mixed with the other streams to start the foaming reaction.

The dressing of the invention has mainly been described with reference to wound dressings, but it will be evident for the skilled in the art that the invention is not limited to wound dressings. Thus a medical dressing of the invention may be used in wound care, incontinence care, ostomy care and is even not limited to these applications. It could be also used as pad or swob in skin care and cosmetic care in general.

## Example

Materials

Hypol 2002, Dow
Optipol, LMI
Pluronic 6200 PO-PE block copolymer, BASF
Brij 58, Uniquema
Ion exchanged water from internal laboratory supply
Bromophenolblue
Aegis 5772
Lurol 8728
Aqualine PSA 1623

Methods

1. WET-OUT: DRY

- Size : rectangular sample.
  - Requirements: pipet of 1 ml, demineralized water
- Method : Bring a drop of 1 ml on a the horizontal surface of the sample and measure the time (in seconds) that the drop of water needs to penetrate totally in the foam.
- Result : Wet-out dry in seconds/millilitre

2. FLUID CAPACITY

- EN13726-1
- Size : Two rectangular samples (63 x 63 x d mm)
- Requirements :
  - Balance
  - Recipient filled with water (water temperature = 37 ± 0.5°C)
- Method : - weigh the sample dry (G0).
  - immerse the sample in the water during 30 min
  - take the sample horizontally out of the water so that no water drops out (minimum water loss)
  - Weigh the sample immediately (G1)
- Calculation :

$$\text{Ratio g water / g product} = \frac{(G1\text{- }GO)}{G0}$$

3. FLUID CAPACITY AFTER DRAIN

- EN 13726-1
  - Size : Two rectangular samples (63 x 63 x d mm)
  - Requirements : - Balance
    - Recipient filled with water (water temperature = 37 ± 0.5°C)
  - Method : - weigh the sample dry (G0).
    - immerse the sample in the water during 30 min
    - Take the sample out of the water, taking it at one corner, let drop out the excess of not bonded water during 30 seconds
    - weigh the sample (G1).
  - Calculation :

$$\text{Ratio g water / g product} = \frac{(G1\text{-}GO)}{G0}$$

4. DYNAMIC SHAKE FLASK METHOD

ASTM E2149-01

Description of the method see Standard Test "C" described on p. 19 to 21 of EP-A-0 155 155, which is incorporated herein by way of reference.

5. BROMOPHENOL BLUE TEST

Description of the method see Standard Test "B" described on p. 17 to 19 of EP-A-0 155 155, which is incorporated herein by way of reference.

6. FUNGAL GROWTH TEST

AATCC Test Method 30-1999

Example

A polyurethane foam sheet was produced by mixing Hypol 2002 (100 g), water (85g), Pluronic 6200 (1 g) and Aegis 5772 (1.5 g), by first mixing the three last components and then adding this mixture to the prepolymer during mixing.

The mixture was poured down on silicone paper and transformed with a doctor roll and guiding bars to a thin sheet of desired thickness (5 mm). When the material was foamed, it was dried and cured using microwave technology or a ventilated dry air oven.

Alternatively, three separate streams of respectively the prepolymer Hypol 2002, the aqueous phase of solved surfactant Pluronic 6200 and the antimicrobial agent Aegis 5772 were injected in the dynamix mixer, pouring down this mixture on silicone paper.

The presence of the active agent was demonstrated and quantitatively determined with the bromophenol blue test. The final foam contained 0.6 parts by weight of the active agent.

Extraction tests showed no leaching of the active agent, demonstrating that the active agent was immobilised due to chemical bonding to the foam.

Antimicrobial tests (shake flask method) showed a significant reduction of bacterial contamination.

## Claims

1. An antimicrobially active polymeric flexible material suitable for use in body care applications, such as wound care, incontinence care, ostomy care, skin care and cosmetic care, comprising a polymeric substrate having covalently bonded thereto at least one antimicrobially active compound, **characterised in that** said material is hydrophilic having:

   - a fluid absorption capacity after saturation of the material of higher than 4 g per gram material, preferably higher than 8 g, more preferably higher than 12 g, and most preferably higher than 15 g per gram material;
   - a fluid absorption capacity after draining of the material of higher than 2 g, preferably higher than 6 g, more preferably higher than 10 g, and most preferably higher than 13 g per gram material; and
   - a wet out, lower than 200 s, preferably lower than 100 s, more preferably lower than 50 s and most preferably lower than 10 s.

2. A material according to claim 1, **characterised in that** said antimicrobial active compound comprises a cation and is in particular a quaternary ammonium compound.

3. A material according to claim 1 or 2, **characterised in that** said antimicrobial active compound comprises an apolar end chain.

4. A material according to any one of the claims 1 to 3, **characterised in that** said antimicrobial active compound comprises a reactive group by means of which it is covalently bonded to the polymeric substrate, the reactive group being in particular a siloxane, an isocyanate, an alcohol, an epoxide, or a vinyl or allyl monomer.

5. A material according to any one of the claims 1 to 4, **characterised in that** said antimicrobial active compound is present in said material in an amount of less than 1 part by weight, preferably less than 0.80 parts by weight, more preferably less than 0.60 parts by weight, and most preferably less than 0.40 parts by weight per 100 parts by weight of said material.

6. A material according to any one of the claims 1 to 5, **characterised in that** a hydrophilizing agent is incorporated in the material to improve the hydrophilic and mechanical properties thereof.

7. A material according to any one of the claims 1 to 6, **characterised in that** said polymeric substrate comprises a hydrophilic polyurethane foam.

8. A wound dressing comprising an antimicrobially active polymeric material according to any one of the previous claims.

9. A wound dressing according to claim 8, **characterised in that** it is provided with an adhesive at the wound covering surface to be self adherent.

10. A wound dressing according to claim 9, **characterised in that** said adhesive is a water based polyacrylate based adhesive.

11. A wound dressing according to claim 9 or 10, **characterised in that** said adhesive is applied by dot screen printing.

12. A wound dressing according to any one of the claims 9 to 11, **characterised in that** said adhesive is a water based polyacrylate based adhesive, applied in amounts of between 25 $g/m^2$ and 300 $g/m^2$, preferably between 50 $g/m^2$ and 250 $g/m^2$, and more preferably between 100 $g/m^2$ and 200 $g/m^2$.

13. A method for producing an antimicrobially active polymeric flexible material according to any one of the claims 1 to 7, wherein the polymeric substrate is a hydrophilic polyurethane foam, **characterised in that** it comprises the step of preparing a reaction mixture starting from at least an isocyanate stream, comprising an isocyanate prepolymer, and an aqueous stream comprising at least one surfactant and said antimicrobially active compound, and the step of allowing the reaction mixture to foam and to cure to produce said polyurethane foam.

14. A method according to claim 13, **characterised in that** the material is produced in a continuous process wherein said reaction mixture is spread in a thin layer and the thin layer is dried and cured preferably with microwave technology, a water based adhesive being optionally applied onto said thin layer before the reaction mixture is entirely cured.

European Patent Office

# EUROPEAN SEARCH REPORT

Application Number
EP 03 07 6909

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | US 2002/177828 A1 (BATICH CHRISTOPHER D ET AL) 28 November 2002 (2002-11-28)<br>* paragraph [0048] *<br>* claims 1,2,4,8 *<br>--- | 1-14 | A61L15/46<br>A61L15/26<br>A61L15/42 |
| A | US 5 302 385 A (KHAN MOHAMMED A ET AL) 12 April 1994 (1994-04-12)<br>* column 1, line 57 - column 2, line 21 *<br>* claims 1,8,10 *<br>--- | 1-14 | |
| A,D | US 4 631 297 A (BATTICE DAVID R ET AL) 23 December 1986 (1986-12-23)<br>* column 2, line 8 - column 3, line 4 *<br>----- | 1-14 | |
| | | | TECHNICAL FIELDS SEARCHED (Int.Cl.7) |
| | | | A61L |

The present search report has been drawn up for all claims

| Place of search | Date of completion of the search | Examiner |
|---|---|---|
| THE HAGUE | 2 December 2003 | Heck, G |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 03 07 6909

This annex lists the patent family members relating to the patent documents cited in the above-mentioned European search report.
The members are as contained in the European Patent Office EDP file on
The European Patent Office is in no way liable for these particulars which are merely given for the purpose of information.

02-12-2003

| Patent document cited in search report | Publication date | Patent family member(s) | Publication date |
|---|---|---|---|
| US 2002177828 A1 | 28-11-2002 | WO 03039602 A2 | 15-05-2003 |
| | | AU 2169500 A | 26-06-2000 |
| | | CA 2353436 A1 | 15-06-2000 |
| | | EP 1156766 A1 | 28-11-2001 |
| | | JP 2003527145 T | 16-09-2003 |
| | | CN 1348346 T | 08-05-2002 |
| | | WO 0033778 A1 | 15-06-2000 |
| US 5302385 A | 12-04-1994 | NONE | |
| US 4631297 A | 23-12-1986 | AU 568927 B2 | 14-01-1988 |
| | | AU 3977085 A | 19-09-1985 |
| | | CA 1245400 A1 | 22-11-1988 |
| | | DE 3577447 D1 | 07-06-1990 |
| | | EP 0155155 A2 | 18-09-1985 |
| | | JP 1778858 C | 13-08-1993 |
| | | JP 4061017 B | 29-09-1992 |
| | | JP 60217247 A | 30-10-1985 |

EPO FORM P0459

For more details about this annex : see Official Journal of the European Patent Office, No. 12/82